# EUROPEAN PATENT APPLICATION

(11) **EP 3 842 518 A1**
(43) Date of publication of application: **30.06.2021**
(21) Application number: 19852380.5
(22) Date of filing: 06.08.2019
(51) Int. Cl.: C12N 5/00, C12M 1/00, C12M 3/00, C12M 3/06

(54) **CELL CULTURE METHOD AND CELL CULTURE DEVICE**

(30) Priority: 20.08.2018 JP 2018154089
(71) Applicant: FUJIFILM Corporation, Tokyo 106-8620 (JP)
(72) Inventor: INADA, Atsushi, Ashigarakami-gun, Kanagawa 258-8577 (JP); TAKAYAMA, Hidetoshi, Ashigarakami-gun, Kanagawa 258-8577 (JP)
(74) Representative: Klunker IP Patentanwälte PartG mbB
(86) International application number: PCT/JP2019/030860
(87) International publication number: WO 2020/039911

(57) **Abstract**

A cell culture method includes a classification step of separating components of a cell suspension containing cell aggregates transferred from a culture vessel according to a size; a first collection step of collecting cell aggregates having relatively small sizes separated in the classification step, in the culture vessel; a division step of dividing cell aggregates having relatively large sizes separated in the classification step; and a second collection step of collecting the cell aggregates divided in the division step, into the culture vessel or a collection vessel different from the culture vessel.

## Description

### Technical Field

Priority is claimed on Japanese Patent Application No. 2018-154089, filed on August 20, 2018, the content of which is incorporated herein by reference.

A disclosed technique relates to a cell culture method and a cell culture apparatus.

### Related Art

Regarding a cell culture apparatus, for example, JP2016-131538A discloses a cell culture apparatus including a culture vessel, a storage vessel, and a division treatment unit that performs division treatment to divide cell aggregates, and a medium supply unit that supplies a medium into a flow path.

### SUMMARY OF INVENTION

### Technical Problem

In culturing of stem cells such as induced pluripotent stem cells (iPS cells), in a case where sizes of cell aggregates (spheres) generated by suspension culture of cells become excessively large, the cell aggregates may adhere and fuse with each other to cause differentiation of the cells to initiate or cause necrosis of cells in the center of the cell aggregate to occur. Therefore, in order to prevent the sizes of the cell aggregates from becoming excessively large, a division treatment in which the cell aggregates are divided (crushed) into a plurality of cell aggregates having smaller sizes is performed at an appropriate time during the cell culture period.

As a method of dividing the cell aggregates, a method of mechanically dividing the cell aggregates by passing the cell suspension containing cell aggregates through a mesh having a plurality of openings (mesh) has been proposed. In the division treatment using the mesh, the cell aggregates are damaged when colliding with the mesh, and many dead cells are generated. In particular, cell aggregates having relatively small sizes are vulnerable to the division treatment using the mesh. During the culture period, cell aggregates having various sizes are stored in a culture vessel. In a case where the division treatment is indiscriminately performed on the cell aggregates stored in the culture vessel, cell aggregates having relatively small sizes will also be divided. Accordingly, it becomes difficult to suppress the incidence of dead cells.

The disclosed technique provides a cell culture method and a cell culture apparatus which are capable of suppressing generation of dead cells by suppressing execution of a division treatment on cell aggregates having relatively small sizes.

### Solution to Problem

A cell culture method according to the disclosed technique comprises a classification step of separating components of a cell suspension containing cell aggregates transferred from a culture vessel according to a size; a first collection step of collecting cell aggregates having relatively small sizes separated in the classification step, in the culture vessel: a division step of dividing cell aggregates having relatively large sizes separated in the classification step; and a second collection step of collecting the cell aggregates divided in the division step, in the culture vessel or a collection vessel different from the culture vessel. According to the cell culture method according to the disclosed technique, the execution of the division treatment on the cell aggregates having relatively small sizes is suppressed, and thus, it is possible to suppress the generation of dead cells.

The cell culture method according to the disclosed technique may further comprise a disposal step of disposing of components belonging to a class having a smallest size separated in the classification step. Accordingly, it is possible to remove debris such as dead cells from the cell suspension.

The cell culture method according to the disclosed technique may further comprise a mixing step of mixing the cell aggregates with a medium before dividing the cell aggregates in the division step. Accordingly, it is possible to adjust a cell concentration in the cell suspension that has undergone the classification step to an appropriate concentration.

The cell culture method according to the disclosed technique may further comprise: a first culture step of culturing cells collected in the collection vessel in the second collection step, in the collection vessel; and a second culture step of culturing the cells that have undergone the first culture step, in the culture vessel. Accordingly, the cells can be cultured in an environment suitable for a state of the cells, and it is possible to improve a viability and a quality of the cells.

For example, a composition of a medium used in the first culture step may differ from a composition of a medium used in the second culture step. Specifically, a ROCK inhibitor may be added to the medium used in the first culture step, and it is preferable that a ROCK inhibitor is not added to the medium used in the second culture step. In addition, viscosity of the medium used in the first culture step may be lower than viscosity of the medium used in the second culture step.

A cell culture apparatus according to the disclosed technique comprises: a culture vessel storing a cell suspension containing cell aggregates; a classification unit that separates components of the cell suspension containing the cell aggregates according to a size; a division unit that divides the cell aggregates; a flow path connected to the culture vessel, the classification unit, and the division unit; and a controller that controls transfer of the cell suspension through the flow path. The controller transfers the cell suspension stored in the culture vessel to the classification unit, transfers the cell suspension containing cell aggregates having relatively small sizes separated in the classification unit to the culture vessel, transfers the cell suspension containing cell aggregates having relatively large sizes separated in the classification unit to the division unit, and transfers the cell suspension containing the cell aggregates divided in the division unit to a vessel connected to the flow path. According to the cell culture apparatus according to the disclosed technique, the execution of the division treatment on the cell aggregates having relatively small sizes can be suppressed, and thus, it is possible to suppress the generation of dead cells.

The cell culture apparatus according to the disclosed technique may further comprise a waste liquid vessel connected to the flow path. In this case, the controller may transfer components belonging to a class having a smallest size separated in the classification unit to the waste liquid vessel. Accordingly, it is possible to remove debris such as dead cells from the cell suspension.

The controller may transfer the cell suspension containing the cell aggregates divided in the division unit to the culture vessel. In addition, the cell culture apparatus according to the disclosed technique may further comprise a collection vessel connected to the flow path. In this case the controller may transfer the cell suspension containing the cell aggregates divided in the division unit to the collection vessel.

The controller may transfer a cell suspension, which is transferred to the collection vessel and contains cells cultured in the collection vessel, to the culture vessel. Accordingly, the cells can be cultured in an environment suitable for a state of the cells, and it is possible to improve a viability and a quality of the cells.

The cell culture apparatus according to the disclosed technique may further comprise a filtration unit that is connected to the flow path and filters the cell suspension. In this case, the controller may transfer the cell suspension, which is transferred to the collection vessel and contains cells cultured in the collection vessel, to the filtration unit, before transferring the cell suspension to the culture vessel, and transfer the cell suspension filtered in the filtration unit to the culture vessel. Accordingly, it is possible to remove debris such as dead cells from the cell suspension.

### Effects of Invention

According to the disclosed technique, the execution of the division treatment on the cell aggregates having relatively small sizes is suppressed, and thus, it is possible to suppress the generation of dead cells.

### BRIEF DESCRIPTION OF THE DRAWINGS

Fig. 1 is a step flow chart showing an example of a treatment flow in a cell culture method according to an embodiment of the disclosed technique.
Fig. 2 is a diagram showing an example of a configuration of a cell culture apparatus according to the embodiment of the disclosed technique.
Fig. 3A is a cross sectional view showing an example of a configuration of a division unit 16 according to the embodiment of the disclosed technique.
Fig. 3B is a plan view of a mesh according to the embodiment of the disclosed technique.
Fig. 3C is an enlarged view of a portion Y surrounded by a broken line in Fig. 3B.
Fig. 4 is a flowchart showing an example of a flow of treatments performed by a controller according to the embodiment of the disclosed technique, in a case of performing a medium replacement treatment.
Fig. 5 is a flowchart showing an example of a flow of treatments performed by the controller according to the embodiment of the disclosed technique, in a case of performing a division treatment.
Fig. 6 is a step flow chart showing an example of a treatment flow in a cell culture method according to another embodiment of the disclosed technique.
Fig. 7 is a diagram showing an example of a configuration of a cell culture apparatus according to another embodiment of the disclosed technique.
Fig. 8 is a flowchart showing an example of a flow of treatments performed by the controller according to the embodiment of the disclosed technique, in a case of performing the division treatment.

### DESCRIPTION OF EMBODIMENTS

Hereinafter, an example of an embodiment of the disclosed technique will be described with reference to the drawings. In each drawing, the same or equivalent components and parts are designated by the same reference numerals.

### [First embodiment]

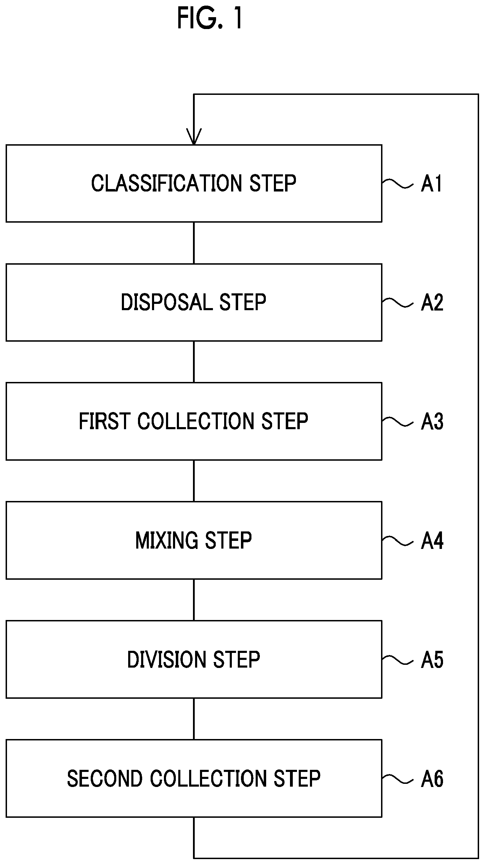

Fig. 1 is a step flow chart showing an example of a treatment flow in a cell culture method according to a first embodiment of the disclosed technique. In the cell culture method according to the present embodiment, proliferative stem cells such as iPS cells, mesenchymal embryo cells, and ES cells are targets to be cultured. In addition, it is assumed that, for example, 1×10⁹ cells or more cells are cultured in a culture vessel in a state of forming a substantially spherical aggregate (sphere) and being suspended in a medium. The cell culture method according to the present embodiment includes a classification step A1, a disposal step A2, a first collection step A3, a mixing step A4, a division step A5, and a second collection step A6.

In the classification step A1, a classification treatment of classifying components contained in the cell suspension transferred from the culture vessel is performed. In the classification step A1 according to the present embodiment, the components contained in the cell suspension are divided into three classes according to sizes thereof. The components belonging to the class having the smallest size include debris such as dead cells having a size, for example, of less than 50 µm and secretions secreted from the cells. The components belonging to the class having the largest size include cell aggregates having a relatively large size (for example, 200 µm or more) (hereinafter, large size spheres). The components belonging to the class having approximately the medium size include cell aggregates having a relatively small size (for example, 50 µm or larger and smaller than 200 µm) (hereinafter, small size spheres).

In the disposal step A2, debris such as dead cells contained in the component belonging to the class having the smallest size, separated from another class components in the classification step is disposed.

In the first collection step A3, the small size spheres separated from another class components in the classification step A1 are collected in the original culture vessel.

In the mixing step A4, a mixing treatment of mixing a cell suspension containing large size spheres separated from another class components in the classification step A1 with a fresh medium is performed. In the classification step A1, the cell aggregates and the medium are separated, and the cell suspension that has undergone the classification step A1 has an excessively high cell concentration. Therefore, the cell concentration in the cell suspension containing the large size spheres is adjusted to an appropriate concentration by mixing the cell suspension containing the large size spheres with the fresh medium.

In the division step A5, a division treatment of dividing the large size spheres separated from another class components in the classification step A1 into cell aggregates having smaller sizes is performed. The division of the cell aggregates in the division step A5 is performed by passing the cell aggregates through a mesh. In culturing of stem cells such as iPS cells, in a case where sizes of cell aggregates generated by suspension culture of cells become excessively large, the cell aggregates may adhere and fuse with each other to cause differentiation of the cells to initiate or cause necrosis of cells in the center of the cell aggregate to occur. It is possible to suppress the size of the cell aggregate from becoming excessively large, by performing the division treatment in the division step A5.

In the second collection step A6, the cell aggregates divided in the division step A5 are collected in the original culture vessel or another collection vessel different from the culture vessel. The collected cells are continuously cultured in the original culture vessel or another collection vessel different from the culture vessel. By the culturing, for example, in a case where an average size of the cell aggregates is grown to a predetermined size in which the division treatment is required, the treatment in each of the steps (A1 to A6) is repeated.

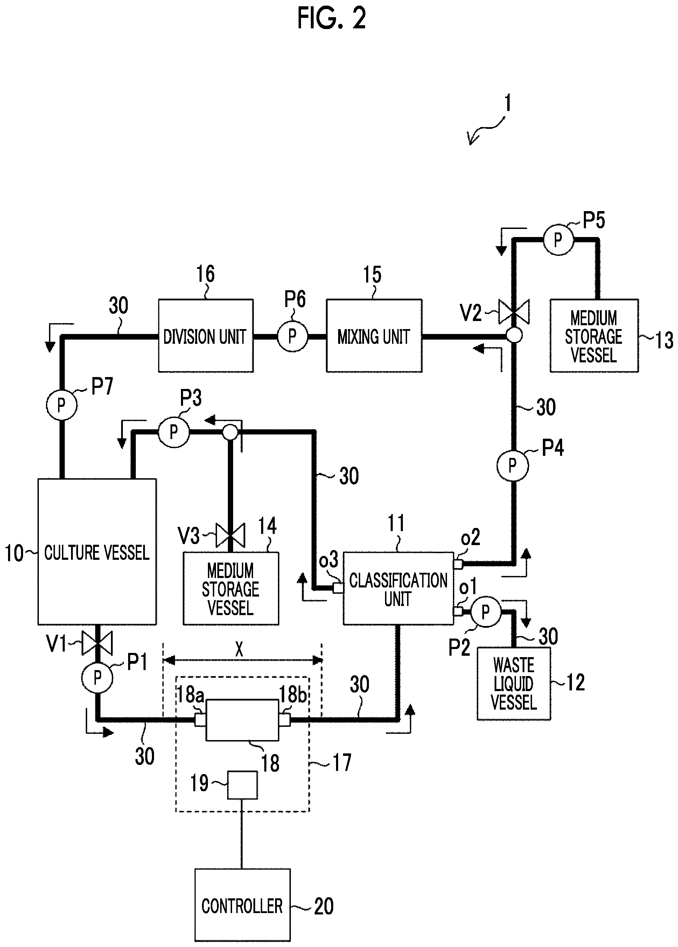

Fig. 2 is a diagram showing an example of a configuration of a cell culture apparatus 1 according to the first embodiment of the disclosed technique, for realizing the cell culture method described above.

The cell culture apparatus 1 is configured to include a culture vessel 10, medium storage vessels 13 and 14, a classification unit 11, a waste liquid vessel 12, a mixing unit 15, a division unit 16, a monitor unit 17, a controller 20, pumps P1 to P7, and valves V1 to V3. The culture vessel 10, the medium storage vessels 13 and 14, the classification unit 11, the waste liquid vessel 12, the mixing unit 15, and the division unit 16 are connected to a flow path 30, respectively.

The culture vessel 10 stores a cell suspension containing cell aggregates including a plurality of cells which are targets to be cultured and a medium. The culture vessel 10 has, for example, a volume capable of storing 1×10⁹ cells or more cells. A form of the culture vessel 10 is not particularly limited, and for example, a glass vessel or a metal vessel can be used as the culture vessel 10. The culture vessel 10 may have, for example, a form of a bag configured to include a gas permeable film. Each of the medium storage vessels 13 and 14 stores a fresh medium.

The classification unit 11 performs the classification treatment in the classification step A1 of the cell culture method according to the present embodiment. That is, the classification unit 11 performs a classification treatment to classify the components contained in the cell suspension into three classes according to sizes thereof. The classification unit 11 causes the components having different sizes from each other separated by the classification treatment to flow out from separate outlets o1, o2, and o3. The outlet o1 through which components belonging to class having the smallest size (mainly debris such as dead cells) flow out is connected to the waste liquid vessel 12 via the flow path 30. The outlet o2 through which components belonging to class having the largest size (mainly large size spheres) flow out is connected to the mixing unit 15 via the flow path 30. The outlet o3 through which components belonging to class having the medium size (mainly small size spheres) flow out is connected to the culture vessel 10 via the flow path 30.

The number of classes of the classifications in the classification unit 11 is variable, and the components contained in the cell suspension can also be classified into, for example, two classes according to sizes thereof. Accordingly, the classification unit 11 also functions as a filtration unit that performs a filtration treatment of removing debris such as dead cells contained in the cell suspension. A known classification device can be used as the classification unit 11. As the classification device configuring the classification unit 11, for example, a device that uses a difference in sedimentation speed in each size of a target to be classified, a device that uses centrifugation, or a device that performs membrane separation using a filter membrane can be used.

The mixing unit 15 performs the mixing treatment in the mixing step A4 of the cell culture method according to the present embodiment. That is, the large size spheres separated in the classification step A1 and the fresh medium transferred from the medium storage vessel 13 are mixed in the mixing unit 15. The mixing unit 15 has a function of stirring an inflowing fluid. The mixing unit 15 may be configured to include a so-called static mixer that does not have a driving unit. For example, the mixing unit 15 can be configured to include a tubular body and a stirring element which is fixedly installed inside the tubular body and forms a spiral flow path inside the tubular body. The mixing unit 15 may be configured to include a stirring device that rotates a stirring blade attached to a rotation shaft, around the rotation shaft.

The division unit 16 performs the division treatment in the division step A5 of the cell culture method according to the present embodiment. That is, the division unit 16 divides the large size spheres separated in the classification unit 11 and mixed with the fresh medium in the mixing unit 15 into cell aggregates having smaller sizes.

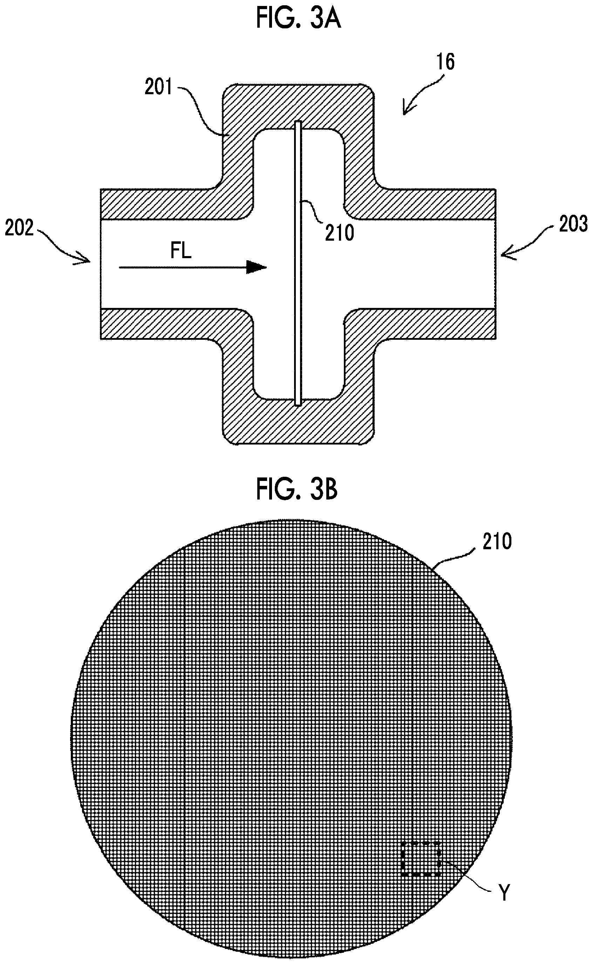

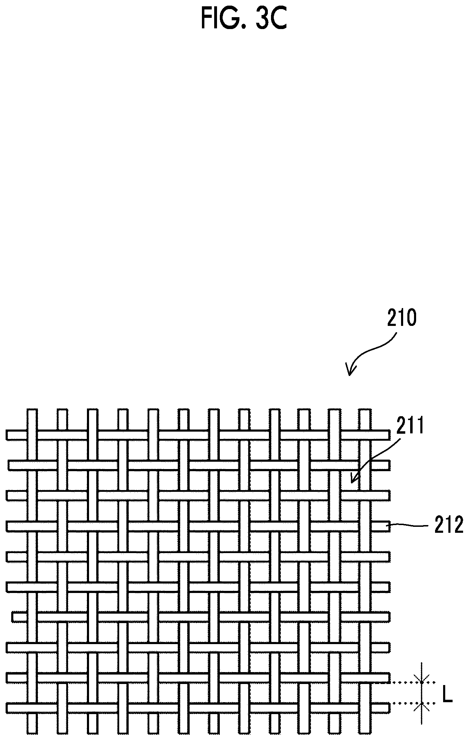

Fig. 3A is a cross-sectional view showing an example of the configuration of the division unit 16. The division unit 16 is configured to include a case 201 having an inlet 202 and an outlet 203, and a mesh 210 provided between the inlet 202 and the outlet 203 inside the case 201. Fig. 3B is a plan view of the mesh 210, and Fig. 3C is an enlarged view of a portion Y surrounded by a broken line in Fig. 3B. The mesh 210 has a plurality of openings (mesh) 211 formed by, for example, plain weaving a plurality of fibrous members 212. The weave of the fibrous member 212 is not limited to plain weave. A material of the fibrous member 212 is not particularly limited, and it is preferable that the fibrous member 212 is made of a material having high corrosion resistance. For example, nylon or stainless steel can be preferably used. The mesh 210 is installed in the case 201 such that a main surface having a plurality of openings 211 extends in a direction intersecting the flow direction FL of the cell suspension. As the cell suspension passes through the mesh 210. the cell aggregates contained in the cell suspension are mechanically divided. A pore diameter L of the mesh 210 of the division unit 16 is, for example, smaller than the average diameter of the cell aggregates before the division treatment, and is determined according to a target size of cell aggregates after the division treatment. As the average diameter of the cell aggregates, it is possible to apply an arithmetic mean of diameters of the spheres when each of the cell aggregates is approximated to a sphere.

The monitor unit 17 is provided in a section X of the flow path 30 between the culture vessel 10 and the classification unit 11. The monitor unit 17 monitors the cell suspension passing through the section X. The monitor unit 17 is configured to include a flow cell 18 and an imaging device 19.

The flow cell 18 is entirely made of a light-transmitting material such as glass or plastic. The flow cell 18 has a first circulation port 18a and a second circulation port 18b communicating with the first circulation port 18a. The imaging device 19 has an imaging field of view set in a region between the first circulation port 18a and the second circulation port 18b of the flow cell 18, and continuously images the cells (cell aggregates) contained in the cell suspension flowing inside the flow cell 18 through the flow cell 18. A plurality of images imaged by the imaging device 19 are transmitted to the controller 20. In addition, the medium contained in the cell suspension flowing inside the flow cell 18 is imaged by the imaging device 19 to evaluate tints, and a pH (hydrogen ion index) of the medium is quantified by collating with a color sample registered in advance.

The flow path 30 is configured to be respectively capable of transferring the cell suspension from the culture vessel 10 to the classification unit 11, transferring the cell suspension from the classification unit 11 to the culture vessel 10, the waste liquid vessel 12, and mixing unit 15, transferring the cell suspension from the mixing unit 15 to the division unit 16, and transferring the cell suspension from the division unit 16 to the culture vessel 10. Further, the flow path 30 is configured to be respectively capable of transferring the medium from the medium storage vessel 13 to the mixing unit 15 and transferring the medium from the medium storage vessel 14 to the culture vessel 10. In addition, The flow path 30 is configured to form a first circulation route in which the cell suspension extracted from the culture vessel 10 returns to the culture vessel 10 via the classification unit 11, the mixing unit 15, and the division unit 16, and a second circulation route in which the cell suspension extracted from the culture vessel 10 returns to the culture vessel 10 via the classification unit 11.

The pumps P1 to P7 and the valves V1 to V3 are appropriately positioned at each position in the flow path 30. The pumps P1 to P7 are driven according to a control signal supplied from the controller 20, and the valves V1 to V3 are opened and closed according to the control signal supplied from the controller 20. The configuration of the flow path 30 and the positioning of the pumps P1 to P7 and the valves V1 to V3 are not limited to those illustrated in Fig. 2, as long as the flow path 30, the pumps P1 to P7. and valves V1 to V3 are configured such that the cell culture method according to the present embodiment can be performed.

The controller 20 controls the transfer of the cell suspension through the flow path 30, by performing a driving control of the pumps P1 to P7 and an opening-and-closing control of the valves V1 to V3 using the control signal.

In a case where cell culture is performed in the culture vessel 10, debris such as dead cells and secretions secreted from the cells are accumulated in the culture vessel 10. Since these debris may adversely affect the viability and a proliferative property of the cells, it is preferable that a medium replacement treatment of regularly (for example, every 24 hours) replacing the medium in the culture vessel 10 with a fresh medium is carried out.

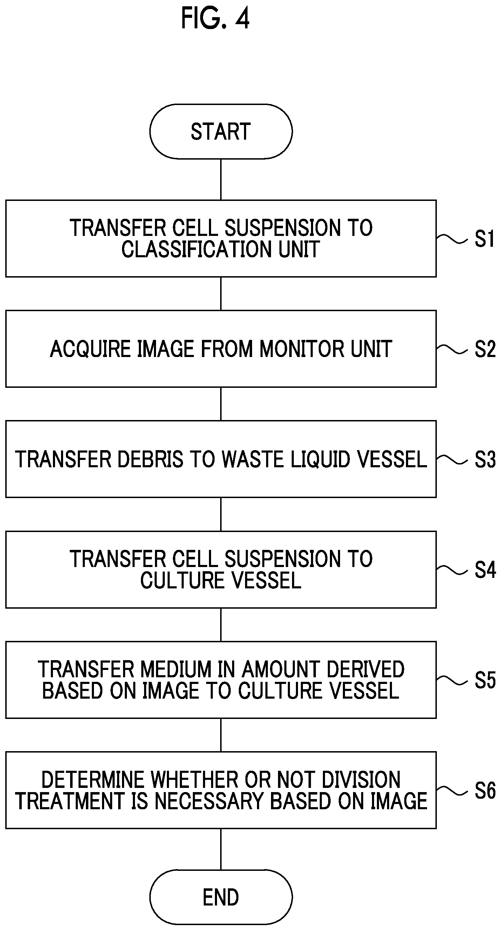

Fig. 4 is a flowchart showing an example of a flow of treatments performed by the controller 20, in a case of performing the medium replacement treatment in the cell culture apparatus 1.

In Step S1, the controller 20 controls the valve V1 to be in an open state and drives the pump P1 to transfer a part of the cell suspension stored in the culture vessel 10 to the classification unit 11. While the cell suspension passes through the section X of the flow path 30, the cells contained in the cell suspension are monitored by the monitor unit 17. That is, the imaging device 19 continuously images the cells (cell aggregates) contained in the cell suspension passing through the flow cell 18. The imaging device 19 images, for example, all cells (cell aggregates) contained in the cell suspension passing through the section X of the flow path 30 at intervals at which imaging can be performed. The imaging device 19 may image some cells (cell aggregates) contained in the cell suspension passing through the section X of the flow path 30.

In Step S2, the controller 20 acquires an image of the cell imaged by the imaging device 19 of the monitor unit 17.

The classification unit 11 functions as a filtration unit and performs a filtration treatment on the cell suspension transferred from the culture vessel 10. That is, the classification unit 11 separates cell aggregates having relatively large sizes and debris such as dead cells having relatively small sizes, contained in the cell suspension transferred from the culture vessel 10. The classification unit 11 causes the cell aggregates having relatively large sizes to flow out from the outlet o3 and cell aggregates having relatively small sizes to flow out from the outlet o1.

In Step S3, the controller 20 drives the pump P2 to transfer the debris such as dead cells flowing out from the outlet o1 to the waste liquid vessel 12.

In Step S4, the controller 20 drives the pump P3 to transfer the cell suspension containing cell aggregates flowing out from the outlet o3 to the culture vessel 10.

In Step S5, the controller 20 drives the pump P3 and controls the valve V3 to be in the open state, so that the fresh medium stored in the medium storage vessel 14 is transferred to the culture vessel 10. Accordingly, the culture vessel 10 is supplemented with a fresh medium. In this case, the controller 20 derives a transfer amount of the medium on the basis of the image or an evaluation result acquired from the monitor unit 17 in Step S2. The controller 20 derives the transfer amount of the medium as follows, for example. That is, the controller 20 derives a current cell concentration in the culture vessel 10 on the basis of the image acquired in Step S2. The current cell concentration can be derived, for example, from a ratio between an integrated value of the individual sizes of the cell aggregates contained in the image acquired in Step S2 and an area of the imaging field of view of the imaging device 19. The controller 20 derives, on the basis of the derived current cell concentration in the culture vessel 10, the amount of the medium to be added in order to bring the cell concentration in the culture vessel 10 to a predetermined concentration, as the transfer amount of the medium from the medium storage vessel 14 to the culture vessel 10.

In Step S6, the controller 20 determines whether or not the division treatment is necessary based on the image acquired from the monitor unit 17 in Step S2. The controller 20 determines whether or not the division treatment is required, for example, as follows. The controller 20 derives an average diameter of the cell aggregates contained in the image acquired from the monitor unit 17 in Step S2, for example. In a case where the derived average diameter is larger than a predetermined size, the controller 20 determines that the division treatment is required, and in a case where the derived average diameter is smaller than the predetermined size, the controller 20 determines that the division treatment is unnecessary. As the average diameter of the cell aggregates, it is possible to apply an arithmetic mean of diameters of the spheres when each of the cell aggregates is approximated to a sphere.

The controller 20 maintains freshness of the medium in the culture vessel 10 at a constant level by repeatedly or continuously performing the treatments of Steps S1 to S6.

In culturing of stem cells such as iPS cells, in a case where sizes of cell aggregates generated by suspension culture of cells become excessively large, the cell aggregates may adhere and fuse with each other to cause differentiation of the cells to initiate or cause necrosis of cells in the center of the cell aggregate to occur. Therefore, in order to prevent the sizes of the cell aggregates from becoming excessively large, it is preferable that the division treatment in which the cell aggregates are divided into a plurality of cell aggregates having smaller sizes is performed at an appropriate time during the cell culture period.

In the cell culture apparatus 1 according to the present embodiment, in a case of performing the medium replacement treatment, the division treatment is performed in a case where it is determined that the division treatment is required in Step S6 (see Fig. 4) of the treatment performed by the controller 20.

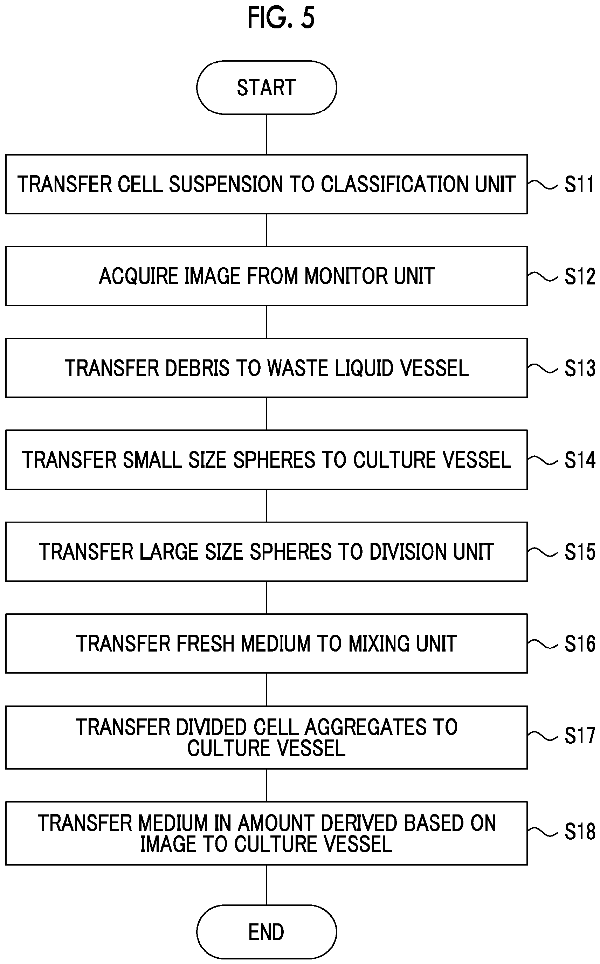

Fig. 5 is a flowchart showing an example of a flow of treatments performed by the controller 20, in a case of performing the division treatment in the cell culture apparatus 1.

In Step S11, the controller 20 controls the valve V1 to be in an open state and drives the pump P1 to transfer a part of the cell suspension stored in the culture vessel 10 to the classification unit 11 from the culture vessel 10. While the cell suspension passes through the section X of the flow path 30, the cells contained in the cell suspension are monitored by the monitor unit 17. That is, the imaging device 19 continuously images the cells (cell aggregates) contained in the cell suspension passing through the flow cell 18.

In Step S12, the controller 20 acquires an image of the cell imaged by the imaging device 19 of the monitor unit 17.

The classification unit 11 performs the classification treatment to classify the components contained in the cell suspension transferred from the culture vessel 10 into three classes according to sizes thereof. That is, the classification unit 11 separates the large size spheres, the small size spheres, and debris such as dead cells, contained in the cell suspension transferred from the culture vessel 10, from each other. Accordingly, the classification step A1 in the cell culture method according to the present embodiment is realized. The classification unit 11 causes the large size spheres to flow out from the outlet o2, the small size spheres to flow out from the outlet o3, and the debris such as dead cells to flow out from the outlet o1.

In Step S13, the controller 20 drives the pump P2 to transfer the debris such as dead cells flowing out from the outlet o1 to the waste liquid vessel 12. Accordingly, the disposal step A2 in the cell culture method according to the present embodiment is realized.

In Step S14, the controller 20 drives the pump P3 to transfer the cell suspension containing the small size spheres flowing out from the outlet o3 to the culture vessel 10. Accordingly, the first collection step A3 in the cell culture method according to the present embodiment is realized. That is, the small size spheres extracted from the culture vessel 10 are collected in the culture vessel 10 without being subjected to the division treatment by the division unit 16.

In Step S15, the controller 20 drives the pumps P4 and P6 to transfer the cell suspension containing the large size spheres flowing out from the outlet o2 to the division unit 16. The cell suspension containing large size spheres is transferred from the classification unit 11 to the division unit 16 via the mixing unit 15.

In Step S16, the controller 20 controls the valve V2 to be in the open state and drives the pump P5, so that the fresh medium stored in the medium storage vessel 13 is transferred to the mixing unit 15. The cell suspension containing large size spheres heading from the classification unit 11 to the division unit 16 merges with the fresh medium transferred from the medium storage vessel 13 in the mixing unit 15, and is mixed and stirred. Accordingly, the mixing step A4 in the cell culture method according to the present embodiment is realized. In the classification unit 11, the cell aggregates and the medium are separated, and the cell suspension that has passed the classification unit 11 has an excessively high cell concentration. Therefore, the cell concentration in the cell suspension containing the large size spheres is adjusted to an appropriate concentration by mixing the cell suspension containing the large size spheres that have passed the classification unit 11 with the fresh medium.

The large size spheres transferred to the division unit 16 are divided into cell aggregates having smaller sizes, by passing through the mesh 210 (see Figs. 3A. 3B. and 3C) of the division unit 16. Accordingly, the division step A5 in the cell culture method according to the present embodiment is realized.

In Step S17, the controller 20 drives the pump P7 to transfer the cell aggregates divided in the division unit 16 to the culture vessel 10. Accordingly, the second collection step A6 in the cell culture method according to the present embodiment is realized. The cells collected in the culture vessel 10 may be continuously cultured in the culture vessel 10.

In Step S18, the controller 20 drives the pump P3 and controls the valve V3 to be in the open state, so that the fresh medium stored in the medium storage vessel 14 is transferred to the culture vessel 10. Accordingly, the culture vessel 10 is supplemented with a fresh medium. In this case, the controller 20 derives the transfer amount of the medium on the basis of the image or the evaluation result acquired from the monitor unit 17 in Step S12. The controller 20 derives the transfer amount of the medium as follows, for example. That is, the controller 20 derives a current cell concentration in the culture vessel 10 on the basis of the image acquired in Step S12. The current cell concentration can be derived, for example, from a ratio between a product of the number and sizes of the cell aggregates contained in the image acquired in Step S12 and an area of the imaging field of view of the imaging device 19. The controller 20 derives, on the basis of the derived current cell concentration in the culture vessel 10, the amount of the medium to be added in order to bring the cell concentration in the culture vessel 10 to a predetermined concentration, as the transfer amount of the medium from the medium storage vessel 14 to the culture vessel 10.

The culture vessel 10 may be supplemented with a medium, using the medium stored in the medium storage vessel 13. In this case, in Step S14, the medium may be transferred to the culture vessel 10 together with the small size spheres. In addition, in Step S16, the culture vessel 10 may be supplemented with a medium, by using the medium to be mixed with the large size spheres.

As described above, according to the cell culture method and the cell culture apparatus 1 according to the embodiment of the disclosed technique, the components of the cell suspension containing the cell aggregates transferred from the culture vessel 10 are classified in the classification unit 11. The large size spheres separated from another class components by the classification are subjected to the division treatment by the division unit 16 and then collected in the culture vessel 10. On the other hand, the small size spheres separated from another class components by the classification are collected in the culture vessel 10 without being subjected to the division treatment.

Here, in the division treatment using the mesh, the cell aggregates are damaged when colliding with the mesh, and many dead cells are generated. In particular, small size spheres are vulnerable to the division treatment using the mesh. The cell aggregates having various sizes are stored in the culture vessel 10. In a case where the division treatment is indiscriminately performed on the cell aggregates stored in the culture vessel 10, the small size spheres will also be divided. Accordingly, it becomes difficult to suppress the incidence of dead cells.

According to the cell culture method and the cell culture apparatus 1 according to the embodiment of the disclosed technique, a division treatment is performed on the large size spheres, while the small size spheres are collected in the culture vessel 10 without being subjected to the division treatment. Accordingly, the generation of dead cells associated with the division treatment can be suppressed, and the cell productivity can be increased. Since the division treatment is originally unnecessary for the small size spheres, it is considered that there is no harmful effect by suppressing the division treatment for the small size spheres.

In addition, in the division treatment using a mesh, loss occurs due to cells that cannot pass through the mesh staying on the mesh. According to the cell culture method and the cell culture apparatus 1 according to the embodiment of the disclosed technique, the division treatment for the small size spheres is avoided, and therefore, it is possible to suppress cell loss associated with the division treatment.

In the cell culture apparatus 1 according to the present embodiment, the case where the cell aggregates divided by the division unit 16 are collected in the culture vessel 10 has been exemplified, but the present invention is not limited to the aspect. For example, a collection vessel different from the culture vessel 10 may be provided, and the cell aggregates divided by the division unit 16 may be collected in the collection vessel.

### [Second Embodiment]

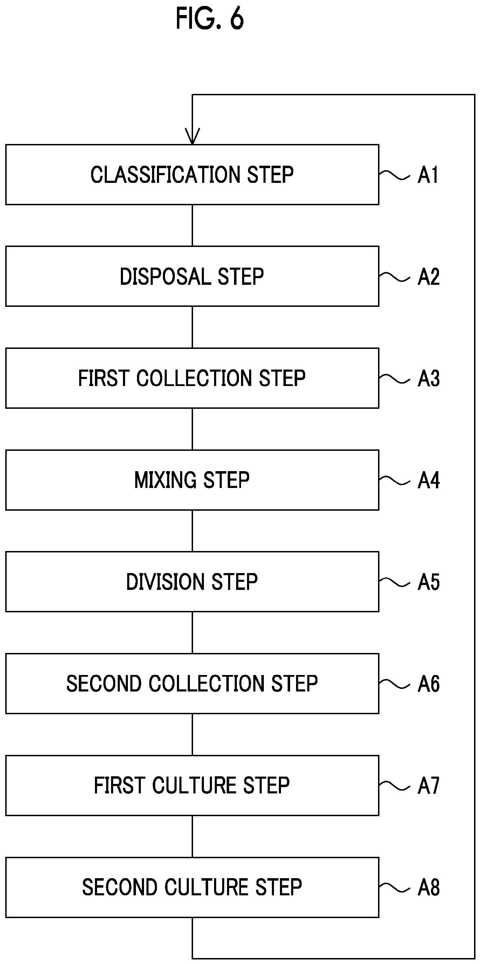

Fig. 6 is a step flow chart showing an example of a treatment flow in a cell culture method according to a second embodiment of the disclosed technique. In the cell culture method according to the second embodiment, a second collection step A6 is different from the second collection step A6 in the cell culture method according to the first embodiment described above. The cell culture method according to the second embodiment further includes a first culture step A7 and a second culture step A8. The classification step A1, the disposal step A2, the first collection step A3, the mixing step A4, and the division step A5 in the cell culture method according to the second embodiment are the same as those in the culture method according to the first embodiment described above. Therefore, duplicate description will be omitted.

In the second collection step A6 according to the second embodiment, the cell aggregates divided in the division step A5 are collected in the original culture vessel or another collection vessel.

In the first culture step A7, the cells collected in the collection vessel are cultured in the collection vessel. That is, the cells damaged due to the division treatment in the division step are cultured in the collection vessel.

In the second culture step A8, the cells that have undergone the first culture step A7 are cultured in the original culture vessel. That is, the cells recovered from the damage by going through the first culture step A7 are transferred to the original culture vessel and then cultured in the culture vessel.

The culture period in the first culture step A7 is, for example, a period (for example, several hours to 1 day) required for recovery from damage due to the division treatment. The culture period in the second culture step A8 is, for example, a period (for example, approximately 5 days) required for the cell aggregate to grow to a size in which the division treatment is required. By the culturing in the second culture step A8. for example, in a case where an average size of the cell aggregates in the culture vessel is grown to a predetermined size in which the division treatment is required, the treatment in each of the steps (A1 to A8) is repeated.

A composition of a medium used in the first culture step A7 may differ from a composition of a medium used in the second culture step A8. A cell damaged due to the division treatment may induce cell death in itself. The cell death that occurs after the division treatment is a factor that reduces cell productivity. It is known that the cell death that occurs after the division treatment can be suppressed by inhibiting an action of intracellular phosphoenzyme called ROCK. Therefore, a ROCK inhibitor that inhibits the action of the ROCK may be added to the medium used in the first culture step A7 performed immediately after the division treatment. An effect of suppressing the cell death that occurs after the division treatment can be expected by adding a ROCK inhibitor to the medium used in the first culture step A7. On the other hand, since spontaneous cell death by cells is a necessary event in the cell growth process, it is preferable to suppress the disadvantages of artificially inhibiting the spontaneous cell death. Therefore, it is preferable that the medium used in the second culture step A8 performed after recovering from the damage due to the division treatment does not contain the ROCK inhibitor.

In addition, since the cell aggregates collected in the collection vessel are reduced in size by the division treatment, viscosity of the medium required to keep the cell aggregates stored in the collection vessel in a suspended state in the medium is relatively low. On the other hand, the average size of the cell aggregates in the culture vessel 10 in which the cells that have undergone the first culture step A7 are stored is larger than the average size of the cell aggregates in the collection vessel. Therefore, the viscosity of the medium required to keep the cell aggregates stored in the culture vessel in a suspended state in the medium is relatively high. Therefore, the viscosity of the medium used in the first culture step A7 may be lower than viscosity of the medium used in the second culture step A8.

In this manner, by making the composition of the medium used in the first culture step A7 and the composition of the medium used in the second culture step A8 different from each other, the culturing suitable for the cell state can be performed in each culture step.

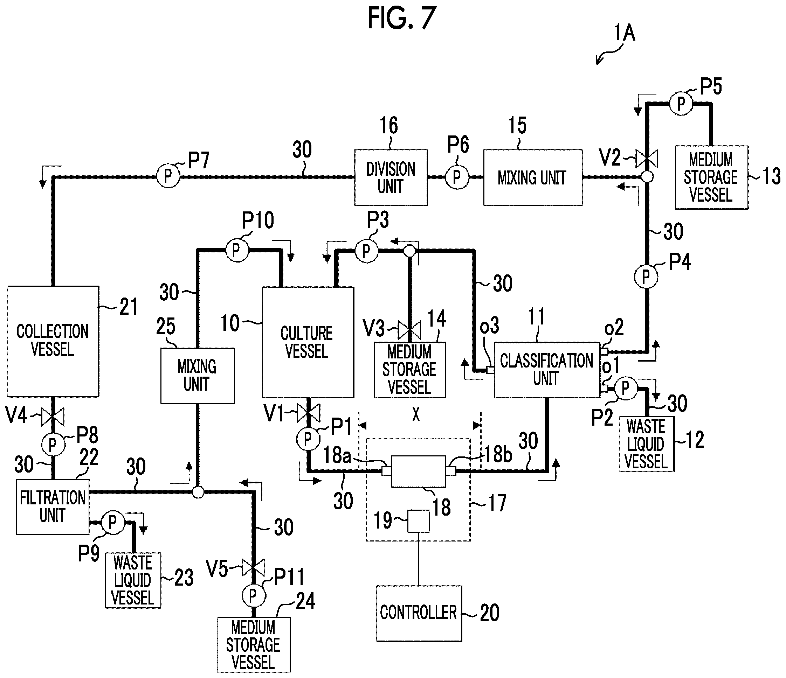

Fig. 7 is a diagram showing an example of a configuration of a cell culture apparatus 1A according to the second embodiment of the disclosed technique, for realizing the cell culture method described above. The cell culture apparatus 1A further includes, in addition to the configuration of the cell culture apparatus 1 according to the first embodiment (see Fig. 2) described above, a collection vessel 21, a filtration unit 22, a waste liquid vessel 23, and a medium storage vessel 24, and a mixing unit 25 which are connected to the flow path 30 are further included.

The collection vessel 21 stores the cell suspension containing the cell aggregates and the medium after the division treatment by the division unit 16. A form of the collection vessel 21 is not particularly limited, and for example, a glass vessel or a metal vessel can be used as the collection vessel 21. The collection vessel 21 may have, for example, a form of a bag configured to include a gas permeable film. The volume of the collection vessel 21 may be equal to the volume of the culture vessel 10 or smaller than the volume of the culture vessel 10.

The filtration unit 22 performs a filtration treatment on the cell suspension to separate the cell suspension into the cell aggregates contained in the cell suspension and the debris such as dead cells. The filtration treatment in the filtration unit 22 may be performed by, for example, membrane separation using a filtration membrane. In a case where the filtration treatment in the filtration unit 22 is performed by the membrane separation, the membrane separation method is preferably a tangential flow method in which damage to cells is relatively small. In the filtration unit 22, the separated debris such as dead cells is stored in the waste liquid vessel 23. A fresh medium is stored in the medium storage vessel 24.

The mixing unit 25 performs a mixing treatment of mixing the cell suspension from which debris such as dead cells has been removed in the filtration unit 22 and the fresh medium transferred from the medium storage vessel 24. Since the cell concentration of the cell suspension subjected to the filtration treatment in the filtration unit 22 is excessively high, the cell concentration in the filtration-treated cell suspension is adjusted to an appropriate concentration by mixing the filtration-treated cell suspension and the fresh medium. The mixing unit 25 may have the same configuration as that of the mixing unit 15, and may be configured to include, for example, a static mixer.

The flow path 30 is configured to be respectively capable of transferring the cell suspension from the culture vessel 10 to the classification unit 11, transferring the cell suspension from the classification unit 11 to the culture vessel 10, the waste liquid vessel 12, and mixing unit 15, transferring the cell suspension from the mixing unit 15 to the division unit 16, transferring the cell suspension from the division unit 16 to the collection vessel 21, transferring the cell suspension from the collection vessel 21 to the filtration unit 22, transferring the cell suspension from the filtration unit 22 to the waste liquid vessel 23 and the mixing unit 25, and transferring the cell suspension from the mixing unit 25 to the culture vessel 10. Further, the flow path 30 is configured to be respectively capable of transferring the medium from the medium storage vessel 13 to the mixing unit 15, transferring the medium from the medium storage vessel 14 to the culture vessel 10, and transferring the medium from the medium storage vessel 24 to the mixing unit 25. In addition, The flow path 30 is configured to form a first circulation route in which the cell suspension extracted from the culture vessel 10 returns to the culture vessel 10 via the classification unit 11, the mixing unit 15, the division unit 16, the collection vessel 21, the filtration unit 22, and the mixing unit 25 and a second circulation route in which the cell suspension extracted from the culture vessel 10 returns to the culture vessel 10 via the classification unit 11.

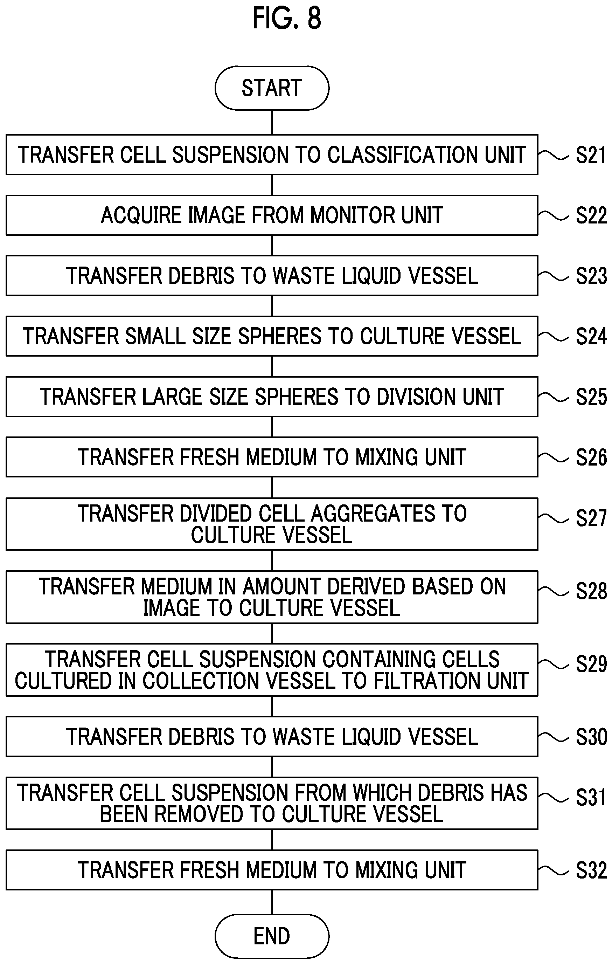

Fig. 8 is a flowchart showing an example of a flow of treatments performed by the controller 20, in a case of performing the division treatment in the cell culture apparatus 1A. Note that the treatments in steps S21 to S26 and S28 of the flowchart shown in Fig. 8 are the same as those in steps S11 to S16 and S18 of the flowchart shown in Fig. 5, and thus description thereof will be omitted.

In Step S27, the controller 20 drives the pump P7 to transfer the cell suspension containing the cell aggregates divided in the division unit 16 to the collection vessel 21. Accordingly, the second collection step A6 in the cell culture method according to the present embodiment is realized. The cells contained in the cell suspension transferred to the collection vessel 21 are continuously cultured in the collection vessel 21. Accordingly, the first culture step A7 in the cell culture method according to the present embodiment is realized.

In a case where a predetermined period (for example, several hours to one day) has elapsed since the start of the first culture step A7, in Step S29, the controller 20 controls the valve V4 to be in an open state and drives the pump P8 to transfer the cell suspension stored in the collection vessel 21 to the filtration unit 22.

The filtration unit 22 performs the filtration treatment on the cell suspension transferred from the collection vessel 21. That is, the filtration unit 22 separates cell aggregates having relatively large sizes and debris such as dead cells having relatively small sizes, contained in the cell suspension transferred from the collection vessel 21.

In Step S30, the controller 20 drives the pump P9 to transfer the debris such as dead cells from the filtration unit 22 to the waste liquid vessel 23.

In Step S31, the controller 20 drives the pump P10 to transfer the cell suspension, from which the debris such as dead cells have been removed, to the culture vessel 10. The cell suspension is transferred from the filtration unit 22 to the culture vessel 10 via the mixing unit 25.

In Step S32, the controller 20 controls the valve V5 to be in the open state and drives the pump P11, so that the fresh medium stored in the medium storage vessel 24 is transferred to the mixing unit 25. The filtration-treated cell suspension heading from the filtration unit 22 to the culture vessel 10 merges with the fresh medium transferred from the medium storage vessel 24 in the mixing unit 25, and is mixed and stirred. Since the cell concentration of the cell suspension subjected to the filtration treatment in the filtration unit 22 is excessively high, the cell concentration in the filtration-treated cell suspension is adjusted to an appropriate concentration by mixing the filtration-treated cell suspension and the fresh medium.

The cells contained in the cell suspension transferred to the culture vessel 10 are continuously cultured in the culture vessel 10. Accordingly, the second culture step A8 in the cell culture method according to the present embodiment is realized.

Specifically, a ROCK inhibitor may be added to the medium used in the first culture step A7 performed in the collection vessel 21, and it is preferable that the ROCK inhibitor is not added to the medium used in the second culture step A8 performed in the culture vessel 10. In addition, viscosity of the medium used in the first culture step A7 may be lower than viscosity of the medium used in the second culture step A8.

According to the cell culture method and the cell culture apparatus 1 according to the second embodiment of the disclosed technique, the divided cell aggregates are collected in the collection vessel 21 different from the culture vessel 10 and cultured in the collection vessel 21 (first culture step). The cells cultured in the collection vessel 21 are transferred to the culture vessel 10 and cultured in the culture vessel 10 after the elapse of a predetermined period (second culture step).

In this manner, the first culture step in which cells damaged due to the division treatment are targets to be cultured and the second culture step in which cells recovered from the damage due to the division treatment are targets to be cultured are performed in different vessels. Accordingly, it is possible to culture the cells in an environment suitable for the state of the cells. Accordingly, it possible to improve viability and quality of cells. For example, a composition of a medium used in the first culture step A7 can be made different from a composition of a medium used in the second culture step A8.

## Claims

1. A cell culture method comprising:
a classification step of separating components of a cell suspension containing cell aggregates transferred from a culture vessel according to a size;
a first collection step of collecting cell aggregates having relatively small sizes separated in the classification step, in the culture vessel;
a division step of dividing cell aggregates having relatively large sizes separated in the classification step; and
a second collection step of collecting the cell aggregates divided in the division step, in the culture vessel or a collection vessel different from the culture vessel.

2. The cell culture method according to claim 1, further comprising:
a disposal step of disposing of components belonging to a class having a smallest size separated in the classification step.

3. The cell culture method according to claim 1 or 2, further comprising:
a mixing step of mixing the cell aggregates with a medium before dividing the cell aggregates in the division step.

4. The cell culture method according to any one of claims 1 to 3, further comprising:
a first culture step of culturing cells collected in the collection vessel in the second collection step, in the collection vessel; and
a second culture step of culturing the cells that have undergone the first culture step, in the culture vessel.

5. The cell culture method according to claim 4,
wherein a composition of a medium used in the first culture step differs from a composition of a medium used in the second culture step.

6. The cell culture method according to claim 5,
wherein a ROCK inhibitor is added to the medium used in the first culture step, and a ROCK inhibitor is not added to the medium used in the second culture step.

7. The cell culture method according to claim 5 or 6,
wherein viscosity of the medium used in the first culture step is lower than viscosity of the medium used in the second culture step.

8. A cell culture apparatus comprising:
a culture vessel storing a cell suspension containing cell aggregates;
a classification unit that separates components of the cell suspension containing the cell aggregates according to a size;
a division unit that divides the cell aggregates;
a flow path connected to the culture vessel, the classification unit, and the division unit; and
a controller that controls transfer of the cell suspension through the flow path,
wherein the controller
transfers the cell suspension stored in the culture vessel to the classification unit,
transfers the cell suspension containing cell aggregates having relatively small sizes separated in the classification unit to the culture vessel,
transfers the cell suspension containing cell aggregates having relatively large sizes separated in the classification unit to the division unit, and
transfers the cell suspension containing the cell aggregates divided in the division unit to a vessel connected to the flow path.

9. The cell culture apparatus according to claim 8, further comprising:
a waste liquid vessel connected to the flow path,
wherein the controller transfers the components belonging to a class having a smallest size separated in the classification unit to the waste liquid vessel.

10. The cell culture apparatus according to claim 8 or 9,
wherein the controller transfers the cell suspension containing the cell aggregates divided in the division unit to the culture vessel.

11. The cell culture apparatus according to any one of claims 8 to 10, further comprising:
a collection vessel connected to the flow path,
wherein the controller transfers the cell suspension containing the cell aggregates divided in the division unit to the collection vessel.

12. The cell culture apparatus according to claim 11,
wherein the controller transfers the cell suspension, which is transferred to the collection vessel and contains cells cultured in the collection vessel, to the culture vessel.

13. The cell culture apparatus according to claim 11 or 12, further comprising:
a filtration unit that is connected to the flow path and filters the cell suspension,
wherein the controller
transfers the cell suspension, which is transferred to the collection vessel and contains cells cultured in the collection vessel, to the filtration unit, before transferring the cell suspension to the culture vessel, and
transfers the cell suspension filtered in the filtration unit to the culture vessel.
